(19) Europäisches Patentamt
European Patent Office
Office européen des brevets

(11) Publication number: **0 252 524 A2**

# (12) EUROPEAN PATENT APPLICATION

(21) Application number: **87110022.8**

(22) Date of filing: **10.07.87**

(51) Int. Cl.4: **C07C 103/34**, C07D 213/40, C07D 213/64, C07D 213/65, C07D 213/68, C07D 239/42, C07D 239/47, C07D 239/52, A61K 31/16, A61K 31/44, A61K 31/505

(30) Priority: **11.07.86 US 884803**

(43) Date of publication of application: **13.01.88 Bulletin 88/02**

(84) Designated Contracting States: **AT BE CH DE ES FR GB GR IT LI LU NL SE**

(71) Applicant: **WARNER-LAMBERT COMPANY**
**201 Tabor Road**
**Morris Plains New Jersey 07950(US)**

(72) Inventor: **Hoefle, Milton Louis**
**1020 Belmont**
**Ann Arbor Michigan 48104(US)**
Inventor: **Holmes, Ann**
**4641 Wylie Road**
**Dexter Michigan 48130(US)**

(74) Representative: **Mansmann, Ivo et al**
**c/o Gödecke AG - Patentabteilung Postfach 569 - Mooswaldallee 1-9**
**D-7800 Freiburg(DE)**

## (54) Aryl- and aralkylamide- derivatives of Omega- (substituted-phenyloxy)-alkanoic acids as inhibitors of acyl-coa: cholesterol acyltransferase and pharmaceuticals containing them.

(57) Substituted anilide-, substituted 5-aminopyrimidinyl amide-and pyridinylalkyl amide-derivatives of Ω-(substituted-phenyl)oxyalkanoic acids of the general formula I

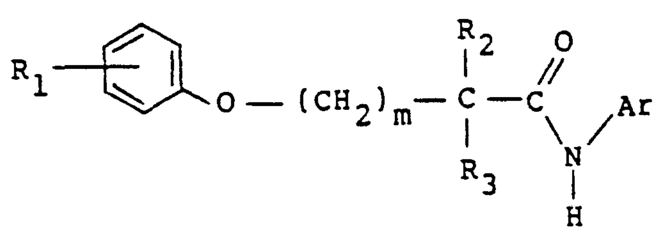

(I),

are potent inhibitors of the enzyme acyl-CoA:cholesterol acyltransferase and are thus useful agents for inhibiting the intestinal absorption of cholesterol.

EP 0 252 524 A2

# ARYL-AND ARALKYLAMIDE - DERIVATIVES OF Ω -(SUBSTITUTED-PHENYLOXY)-ALKANOIC ACIDS AS INHIBITORS OF ACYL-COA:CHOLESTEROL ACYLTRANSFERASE AND PHARMACEUTICALS CONTAINING THEM

This invention relates to chemical compounds having pharmacological activity, to pharmaceutical compositions which include these compounds, and to their use for the preparation of pharmaceuticals. More particularly, this invention concerns certain arylamide-and aralkylamide-derivatives of Ω-(substituted-phenyloxy)alkanoic acid which inhibit acyl-coenzyme A:cholesterol acyltransferase (ACAT), pharmaceutical compositions containing these compounds, and their use for the preparation of pharmaceuticals inhibiting intestinal absorption of cholesterol.

In recent years the role which elevated blood plasma levels of cholesterol plays in pathological conditions in man has received much attention. Deposits of cholesterol in the vascular system have been indicated as causative of a variety of pathological conditions including coronary heart disease.

Initially, studies of this problem were directed toward finding therapeutic agents which would be effective in lowering total serum cholesterol levels. However, cholesterol is transported in the blood in the form of complex particles consisting of a core of cholesteryl esters plus triglycerides and an exterior consisting primarily of phospholipids and a variety of types of protein which are recognized by specific receptors. It is now known that cholesterol is carried to the sites of deposit in blood vessels in the form of low density lipoprotein cholesterol (LDL cholesterol) and away from such sites of deposit by high density lipoprotein cholesterol (HDL cholesterol).

Following these discoveries, the search for therapeutic agents which control serum cholesterol turned to finding compounds which are more selective in their action; that is, agents which are effective in elevating the blood serum levels of HDL cholesterol and/or lowering the levels of LDL cholesterol. While such agents are efective in moderating the levels of serum cholesterol, they have little or no effect on controlling the initial absorption of dietary cholesterol in the body through the intestinal wall.

In intestinal mucosal cells, dietary cholesterol is absorbed as free cholesterol which must be esterified by the action of the enzyme acyl-CoA: cholesterol acyltransferase (ACAT) before it can be packaged into the chylomicrons which are then released into the blood stream. Thus, therapeutic agents which effectively inhibit the action of ACAT prevent the intestinal absorption of dietary cholesterol into the blood stream or the reabsorption of cholesterol which has been previously released into the intestine through the body's own regulatory action.

The present invention provides a class of compounds with ACAT inhibitory activity having the general structure of formula I

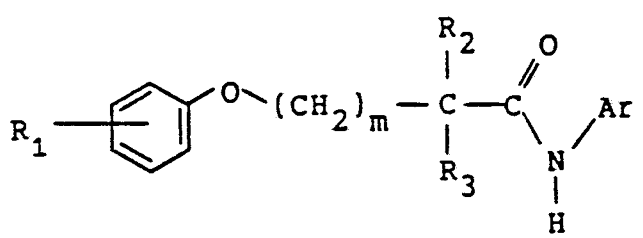

(I),

where $R_1$ is straight or branched alkyl of from one to nine carbon atoms, cycloalkyl of from three to six carbon atoms, or alkyloxy of from one to six carbon atoms.

m is an integer of from one to six.

$R_2$ and $R_3$ are independently selected from hydrogen, straight or branched alkyl of from one to three carbon atoms, or, when taken together with the carbon atom to which they are attached, form a cyclopropyl ring.

Ar is

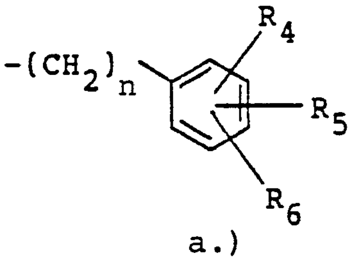, 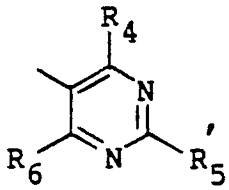, 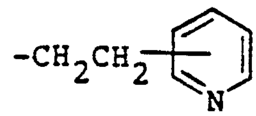,

a.) b.) c.)

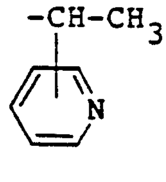, or 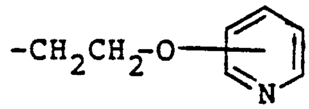

d.) e.)

where n is zero or one and $R_4$, $R_5$ and $R_6$ are independently hydrogen, straight or branched alkyl of from one to three carbon atoms, straight or branched alkyloxy of from one to three carbon atoms, F, Cl, Br, or carboalkoxy of from two to six carbon atoms with the proviso that when Ar is

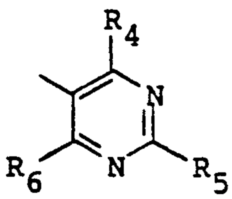,

$R_4$, $R_5$, and $R_6$ may not be F, Cl, or Br.

The compounds of the present invention comprise a class of arylamide-or aralkylamide-derivatives of Ω (substituted-phenyloxy)alkanoic acids. The phenyloxy group of these compounds is substituted in the 2-, 3-, or 4-position with an alkyl, cycloalkyl, or alkyloxy substituent. Preferred compounds of this invention are those where the phenyloxy group is substituted with alkyl, most preferably, 1,1-dimethylethyl.

By the term "alkyl" as used throughout this specification and the appended claims is meant a branched or unbranched hydrocarbon grouping derived from a saturted hydrocarbon by removal of a single hydrogen atom.

The term "cycloalkyl" is meant to denote a substituent derived from a saturated carbocyclic ring by removal of a single hydrogen atom.

By the term "alkyloxy" is meant an alkyl group, as defined above, attached to the parent molecular moiety through an oxygen atom.

The substituted-phenoxy moiety of the compounds of this invention is attached to an alkylene chain which, in turn, is attached at its end furthest from the phenoxy group, to a carbon atom which is substituted with one or two alkyl groups containing independently from one to three carbon atoms. Alternatively, this carbon atom may form, together with its substituent groups, part of a three-membered ring. The preferred compounds of this invention are those in which this methylene group is substituted with two methyl groups.

The phenoxyalkyl group of compounds of this invention is attached in turn to a carboxamido group, i.e.

$-\overset{\overset{\displaystyle O}{\|}}{C}-NH-$,

in which the nitrogen atom is substituted with an aryl or aralkyl group. The aryl group may be mono-, di-or trisubstituted phenyl, mono-, di-, or trisubstituted pyrimidin-5-yl, 1-(2-, 3-, or 4-pyridinyl)ethyl, or 2-(2-, 3-, or 4-pyridinyl)oxyethyl. The aryl substituents may be independently straight or branched alkyl of from one to three carbon atoms, straight or branched alkyloxy of from one to three carbon atoms, F, Cl, Br, or carboalkoxy of from two to six carbon atoms. Preferred compounds of this invention are the substituted anilide compounds.

Illustrative examples of compounds falling within the scope of the present invention are the following:
5-[4-(1,1-dimethylethyl)phenoxy]-N-(2,4-dimethylphenyl)-2,2,-dimethylpentanamide;
N-(2,4-difluorophenyl__-5-[4-(1,1-dimethylethyl)phenoxy]-2,2-dimethylpentanamide;
5-(4-butylphenoxy)-N-(2-methoxy-4-methylphenyl)-2,2-dimethylpentanamide;
5-(4-butylphenoxy)-N-(2-methoxyphenyl)-2,2-dimethylpentanamide;
N -(2,4-dimethoxyphenyl)-5-[4-(1,1-dimethylethyl)phenoxy]-2,2-dimethylpentanamide;
5-(4-butylphenoxy)-2,2-dimethyl-N-(2,4,6-trimethoxyphenyl)-pentanamide;
5-[4-(1,1-dimethylethyl)phenoxy]-2,2-dimethyl-N-(2,4,6-trimethoxyphenyl)pentanamide;
2,2-dimethyl-5-(4-octylphenoxy)-N-(2,4,6-trimethoxyphenyl)pentanamide;
2,2-dimethyl-5-(4-methylphenoxy)-N-[1-(2-pyridinyl)ethyl]pentanamide;
5-(4-butylphenoxy)-2,2-dimethyl-N-[1-(2-pyridinyl)ethyl]pentanamide;
5-[4-(1,1-dimethylethyl)phenoxy]-2,2-dimethyl-N-[1-(2-pyridinyl)ethyl]pentanamide;
5-[4-(1,1-dimethylpropyl)phenoxy]-2,2-dimethyl-N-[1-(2-pyridinyl)ethyl]pentanamide;
5-(4-hexylphenoxy)-2,2-dimethyl-N-[1-(2-pyridinyl)ethyl]pentanamide;
2,2-dimethyl-5-(4-octylphenoxy)-N-[1-(2-pyridinyl)ethyl]pentanamide;
5-(4-butylphenoxy)-2,2-dimethyl-N-[2-(2-pyridinyloxy)ethyl]pentanamide; and
5-(4-butylphenoxy)-N-(4,6-dimethoxy-5-pyrimidinyl)-2,2-dimethylpentanamide.

The compounds of this invention are prepared by the general synthetic methods outlined in the following Reaction Scheme. The starting esters of 2-or 2,2-disubstituted-Ω-haloalkanoic acids, II, and substituted phenols, III are known or, if not previously known, are prepared by known synthetic methods.

REACTION SCHEME

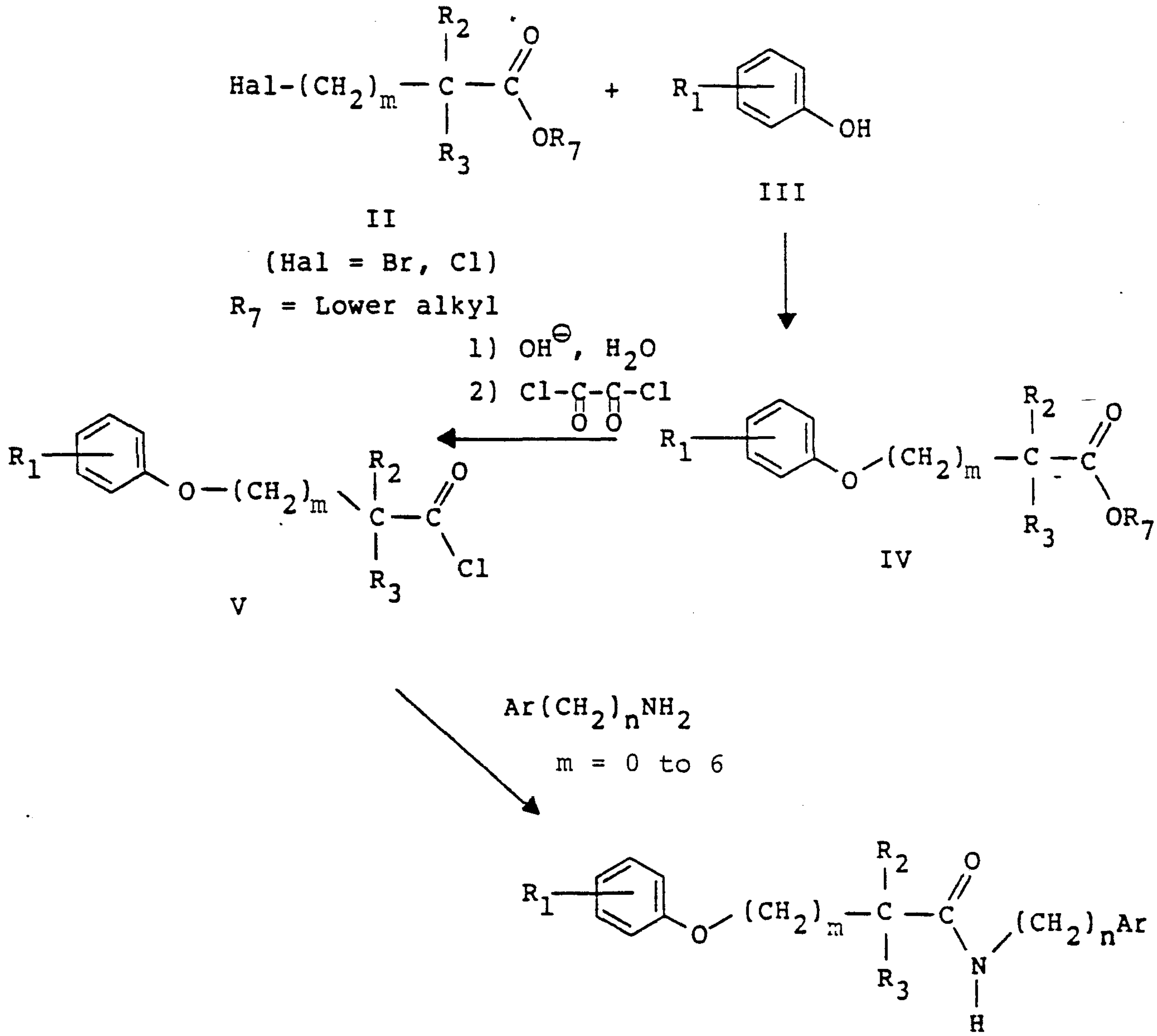


I

The haloacid esters are coupled to the desired substituted phenols by reacting the alkali metal salt of the phenol III, with the haloacid or by direct condensation of the substituted phenol with the haloacid in the presence of a base such as potassium carbonate to produce the Ω-(substituted-phenyl)oxyalkanoic acid IV.

The esters are converted first to their corresponding acids and then to the acid chlorides by conventional means such as hydrolysis followed by reaction with thionyl chloride or oxalyl chloride in an inert solvent such as chloroform.

The amides of this Invention, I, are prepared from the acid chlorides by reaction of the latter with the desired amine in a polar, inert solvent such as tetrahydrofuran in the presence, if desired, of an acid scavenger such as triethylamine. Alternatively, the acids themselves may be condensed with the desired amine in the presence of a suitable coupling reagent such as dicyclohexylcarbodiimide.

As shown by the data presented in the Table below, the compounds of formula I according to the invention are potent inhibitors of the enzyme acyl-COA:cholesterol acyltransferase (ACAT), and are thus effective in inhibiting the esterification and transport of cholesterol across the intestinal cell wall. The compounds of the present invention are thus useful in pharmaceutical formulations for the inhibition of intestinal absorption of dietary cholesterol or the reabsorption of cholesterol released into the intestine by normal body action.

The ability of representative compounds of the present invention to inhibit ACAT was measured using an in vitro test more fully described in Field, F. J. and Salone, R. G., Biochemica et Biophysica 712: 557-570 (1982). The test assesses the ability of a test compound to inhibit the acylation of cholesterol by oleic acid by measuring the amount of radio-labeled cholesterol oleate formed from radio-labeled oleic acid in a tissue preparation containing rabbit intestinal microsomes.

The data appear in the Table where they are expressed as $IC_{50}$ values; i.e. the concentration of test compound required to inhibit cholesteryl oleate formation to 50% of control.

For preparing pharmaceutical compositions from the compounds of this invention, inert, pharmaceutically acceptable carriers can be either solid or liquid. Solid form preparations include powders, tablets, dispersible granules, capsules, and cachets.

A solid carrier can be one or more substances which may also act as diluents, flavoring agents, solubilizers, lubricants, suspending agents, binders, or tablet disintegrating agents; it can also be an encapsulating material.

In powders, the carrier is a finely divided solid which is in a mixture with the finely divided active component. In tablets, the active compound is mixed with the carrier having the necessary binding properties in suitable proportions and compacted in the shape and size desired.

Powders and tablets preferably contain between about 5 to about 70% by weight of the active ingredient. Suitable carriers are magnesium carbonate, magnesium stearate, talc, lactose, sugar, pectin, dextrin, starch, tragacanth, methyl cellulose, sodium carboxymethyl cellulose, a low-melting wax, cocoa butter, and the like.

The term "preparation" is intended to include the formulation of the active compound with encapsulating material as a carrier providing a capsule in which the active component (with or without other carriers) is surrounded by a carrier, which is thus in association with it. In a similar manner, cachets are also included.

Tablets, powders, cachets, and capsules can be used as solid dosage forms suitable for oral administration.

Table

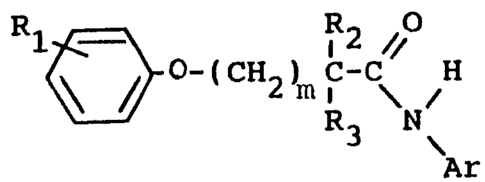

| EXAMPLE | $R_1$ | m | $R_2$ | $R_3$ | Ar | $IC_{50}$ µmoles/liter |
|---|---|---|---|---|---|---|
| 1 | 4-Methyl | 3 | Methyl | Methyl | 1-(2-Pyridinyl)ethyl | 4.8 |
| 2 | 4-Butyl) | 3 | Methyl | Methyl | 2-Methoxyphenyl | 4.3 |
| 3 | 4-Butyl) | 3 | Methyl | Methyl | 2,4-Dimethoxyphenyl | 2.4 |
| 4 | 4-Butyl) | 3 | Methyl | Methyl | 2,4,6-Trimethoxyphenyl | 0.46 |
| 5 | 4-Butyl) | 3 | Methyl | Methyl | 1-(2-Pyridinyl)ethyl | 0.8 |
| 6 | 4-Butyl) | 3 | Methyl | Methyl | 2-(2-Pyridinyloxy) ethyl | 7 |
| 7 | 4-Butyl) | 3 | Methyl | Methyl | 4,6-Dimethyl-5-pyrimidinyl | 9 |
| 8 | 4-(1,1-Dimethyl- ethyl | 3 | Methyl | Methyl | 2,4-Dimethylphenyl | 1.4 |
| 9 | 4-(1,1-Dimethyl- ethyl | 3 | Methyl | Methyl | 2,4-Difluorophenyl | 1.2 |

Table (concluded)

| EXAMPLE | $R_1$ | m | $R_2$ | $R_3$ | Ar | $IC_{50}$ µmoles/liter |
|---|---|---|---|---|---|---|
| 10 | 4-(1,1-Dimethyl-ethyl | 3 | Methyl | Methyl | 2,4,6-Trimethoxyphenyl | 0.044 |
| 12 | 4-(1,1-Dimethyl-ethyl | 3 | Methyl | Methyl | 1-(2-Pyridinyl)ethyl | 0.7 |
| 13 | 4-(1,1-Dimethyl-propyl | 3 | Methyl | Methyl | 1-(2-Pyridinyl)ethyl | 0.16 |
| 15 | 4-Hexyl | 3 | Methyl | Methyl | 1-(2-Pyridinyl)ethyl | 0.7 |
| 16 | 4-Octyl | 3 | Methyl | Methyl | 2,4,6-Trimethoxyphenyl | 0.8 |
| 17 | 4-Octyl | 3 | Methyl | Methyl | 1-(2-Pyridinyl)ethyl | 0.45 |

Liquid form preparations include solutions suitable for oral administration, or suspensions and emulsions suitable for oral administration. Aqueous solutions for oral administration can be prepared by dissolving the active compound in water and adding suitable flavorants, coloring agents, stabilizers, and thickening agents as desired. Aqueous suspensions for oral use can be made by dispersing the finely divided active component in water together with a viscous material such as natural or synthetic gums, resins, methyl cellulose, sodium carboxymethyl cellulose, and other suspending agents known to the pharmaceutical formulation art.

Preferably, the pharmaceutical preparation is in its unit dosage form. In such form, the preparation is divided into unit doses containing appropriate quantities of the active component. The unit dosage form can be a packaged preparation, the package containing discrete quantities of the preparation, for example, packeted tablets, capsules, and powders in vials or ampoules. The unit dosage form can also be a capsule, cachet, or tablet itself, or it can be the appropriate number of any of these packaged forms.

In therapeutic use as agents for the inhibition of intestinal absorption of cholesterol, the compounds utilized in the pharmaceutical method of this invention are administered to the patient as dosage levels of from 500 to 2000 mg per day. For a normal human adult of approximately 70 kg of body weight, this translates into a dosage of from 7 to 30 mg/kg of body weight per day. The specific dosages employed, however, may be varied depending upon the requirements of the patient, the severity of the condition being treated, and the activity of the compound being employed. The determination of optimum dosages for a particular situation is within the skill of the art.

The following preparative examples are provided to enable one skilled in the art to practice the invention, and are illustrative thereof. They are not to be read as limiting the scope of the invention as it is defined by the appended claims. The NMR Spectra (in deutorochloroform) show the chemical shift values (PPM downfield from tetramethylsilane) and the multiplicity.

Example 1

Preparation of 2,2-dimethyl-5-(4-methylphenoxy)-N-[1-(2-pyridinyl)ethyl]pentanamide

Step A - Preparation of 5-(4-methylphenoxy)-2,2-dimethylpentanoic acid methyl ester

A mixture of 21.6 g (0.2 mol) of 4-methylphenol, 44.6 g (0.2 mol) of 5-bromo-2,2-dimethylpentanoic acid methyl ester, 27.6 g (0.2 mol) of potassium carbonate in 300 ml of acetonitrile was stirred and heated under reflux for 24 hours. The mixture was cooled, filtered to remove solids, and concentrated under vacuum. The residue was taken up in diethyl ether and the solution was washed with twice with portions of 1 M aqueous sodium hydroxide, and once with brine solution. The solution was then dried and evaporated under vacuum to yield 46.9 g of 5-(4-methylphenoxy)-2,2-dimethylpentanoic acid methyl ester which was employed in the next step without further purification.

Step B - Preparation of 5-(4-methylphenoxy)-2,2-dimethylpentanoic acid.

The ester from Step A (46.9 g) was mixed with 280 ml of 1 molar aqueous sodium hydroxide solution and 280 ml of methanol. This mixture was stirred and heated under reflux overnight. The mixture was then cooled, concentrated under vacuum, and made acidic with concentrated hydrochloric acid. The resulting solid was taken up in ethyl acetate and the solution washed three times with brine solution, dried, and concentrated under vacuum to yield 41.0 g of 5-(4-methyl-phenoxy)-2,2-dimethylpentanoic acid, mp 95-96°C.

Step C - Preparation of 5-(4-methylphenoxy)-2,2-dimethylpentanoyl chloride.

The acid from Step B (41.0 g) was dissolved in 400 ml of toluene. To this was added slowly, a solution of 43.2 g (0.34 mol) of oxalyl chloride dissolved in 200 ml of toluene. The resulting mixture was stirred at room temperature overnight, and then concentrated under vacuum. The residue was distilled to yield 44.5 g of 5-(4-methyl-phenoxy)-2,2-dimethylpentanoyl chloride, bp 130-135°C at 0.15 torr.

Step D - Preparation of 2,2-dimethyl-5-(4-methylphenoxy)-N-[1-(2-pyridinyl)ethyl]pentanamide

1-(2-Pyridinyl)ethaneamine (3.66 g, 0.33 mol) and triethylamine (3.03 g, 0.03 mol) were dissolved in 100 ml of tetrahydrofuran. To this mixture was slowly added 7.64 g (0.03 mol) of 5-(4-methylphenoxy)-2,2-dimethylpentanoyl chloride dissolved in 100 ml f tetrahydrofuran.

The resulting mixture was stirred at room temperature overnight, filtered, and concentrated under vacuum. The residual oil solidified upon standing overnight to yield 8.7 g of 2,2-dimethyl-5-(4-methylphenoxy)-N-[1-(2-pyridinyl)-ethyl]pentanamide. Recrystallization from n-pentane yielded material melting at 55-57°C.

Analyzed for $C_{21}H_{28}N_2O_2$ :
Calculated : C, 74.08%; H, 8.29%; N, 8.23%;
Found : C, 74.15%, H, 8.2g%; N, 8.50%.

Example 2

Preparation of 5-(4-butylphenoxy)-N-(2-methoxyphenyl)-2,2-dimethylpentanamide

Step A - Preparation of 5-(4-butylphenoxy)-2,2-dimethylpentanoic acid methyl ester

4-Butylphenol (15.0 g (0.1 mol) was dissolved in 200 ml of dry dimethylformamide. To this solution was added, in portions with stirring, 4.8 g (0.1 mol, 50%) of sodium hydride. When the addition was complete, the mixture was stirred for an additional one-half hour. The mixture was then cooled to 15°C and 5-bromo-2,2-dimethylpentanoic acid methyl ester (22.3 g (0.1 mol) dissolved in 100 ml of tetrahydrofuran was slowly added.

This mixture was stirred at room temperature overnight, filtered to remove solids, and then concentrated under vacuum. The residue was taken up in ethyl acetate and washed with successively with portions of 5% aqueous sodium hydroxide solution, 1 molar aqueous hydrochloric acid, and brine. It was then dried and concentrated under vacuum to yield 31.3 g of 5-(4-butylphenoxy)-2,2-dimethylpentanoic acid methyl ester which was used without further purification.

Step B - Preparation of 5-(4-butylphenoxy)-2,2-dimethylpentanoic acid

The ester from step A (31.3 g) was mixed with 150 ml of 1 molar aqueous sodium hydroxide solution and 200 ml of methanol. This mixture was stirred and heated under reflux overnight. The mixture was then cooled, concentrated under vacuum, and acidified with concentrated hydrochloric acid. The solid which separated was taken up in ethyl acetate, and the resulting solution was washed with brine, dried, and evaporated under vacuum to yield 28.4 g of 5-(4-butyl-phenoxy)-2,2-dimethylpentanoic acid, mp 46-48°C.

Step C - Preparation of 5-(4-butylphenoxy)-2,2-dimethylpentanoyl chloride

Employing the method of Example 1, Step C above, the acid was converted by the action of oxalyl chloride to 5-(4-butylphenoxy)-2,2-dimethylpentanoyl chloride (23.4 g, bp 140-145°C at 0.15 torr.

Step D - Employing the method of Example 1, Step D above, the acid chloride was converted, by reaction with 2-methoxybenzeneamine to 10.7 g of 5-(4-butylphenoxy)-N-(2-methoxyphenyl)-2,2-dimethylpentanamide.

Analyzed for $C_{24}H_{33}NO_3$ :
Calculated : C, 75.16%; H, 8.67%; N, 3.65%;
Found : C, 75.45%; H, 8.70%; N, 3.62%.

0.95 (m, 3H), 1.1-1.8 (m, 10H), 1.90 (s, 4H), 2.55 (t, 2H, J = 6.7Hz), 3.95 (s, 5H), 6.8-7.2 (m, 7H), 8.25 (brs, 1H), 7.4 (m, 1H)

Example 3

Preparation of 5-(4-butylphenoxy)-N-(2-methoxy-4-methylphenyl)-2,2-dimethylpentanamide

Employing the methods of Example 2, 12.1 g of 5-(4-butylphenoxy)-N-(2-methoxy-4-methylphenyl)-2,2-dimethylpentanamide were prepared from 2-methoxy-4-methylbenzeneamine and 5-(butylphenoxy)-2,2-dimethylpentanoyl chloride.

Analyzed for $C_{25}H_{35}NO_3$ :

Calculated : C, 75.53%; H, 8.87%; N, 3.52%;

Found : C, 75.65%; H, 8.80%; N, 3.51%.

200 mHz NMR ($CDCl_3$) 0.91 (t, 3H, J = 7Hz), 1.25-1.6 (m, 10H), 1.78 (s, 4H), 2.32 (s, 3H), 2.53 (t, 2H, J = 6.6Hz), 3.86 (s, 3H), 3.90 (m, 2H), 6.7-7.1 (m, 6H), 8.04 (brs, 1H), 8.26 (d, 2H, J = 8Hz)

Example 4

Preparation of 5-(4-butylphenoxy)-2,2-dimethyl-N-(2,4,6-trimethoxyphenyl)-pentanamide

Employing the methods of Example 2, 3.4 g of 5-(4-butylphenoxy)-2,2-dimethyl-N-(2,4,6-trimethoxyphenyl)-pentanamide, mp 79-80°C were prepared from 2,4,6-trimethoxybenzeneamine and 5-(butylphenoxy)-2,2-dimethylpentanoyl chloride.

Analyzed for $C_{26}H_{37}NO_5$:

Calculated : C, 70.40%; H, 8.41%; N, 3.16%;

Found: C, 70.64%; H, 8.27%; N, 3.05%.

mp 79-80°C

Recrystallized from Isopropyl ether

Example 5

Preparation of 5-(4-butylphenoxy)-2,2-dimethyl-N-[1-(2-pyridinyl)ethyl]pentanamide

Employing the methods of Example 2, 9.5 g of 5-(4-butylphenoxy)-2,2-dimethyl-N-[1-(2-pyridinyl)ethyl]-pentanamide, bp 199-201°C at 0.15 torr, were prepared from 1-(2-pyridinyl)ethaneamine and 5-(butylphenoxy)-2,2-dimethylpentanoyl chloride.

Analyzed for $C_{24}H_{34}N_2O_2$ :

Calculated : C, 75.35%; H, 8.96%; N, 7.32%;

Found : C, 75.32%; H, 8.84%; N, 7.38%.

Example 6

Preparation of 5-(4-butylphenoxy)-2,2-dimethyl-N-[2-(2-pyridinyloxy)ethyl]pentanamide

Employing the methods of Example 2, 7.7 g of 5-(4-butylphenoxy)-2,2-dimethyl-N-[2-(2-pyridinyloxy)-ethyl]-pentanamide were prepared from 2-(2-pyridinyloxy)ethaneamine and 5-(butylphenoxy)-2,2-dimethyl-pentanoyl chloride.

Analyzed for $C_{24}H_{34}N_2O_3$ :

Calculated : C, 72.33%; H, 8.60%; N, 7.03%;

Found : C, 72.49%; H, 8.59%; N, 6.99%.

BP 200-205°C / 0.05 torr

Example 7

Preparation of 5-(4-butylphenoxy)-N-(4,6-dimethoxy-5-pyrimidinyl)-2,2-dimethylpentanamide

Employing the methods of Example 2, 13.0 g of 5-(4-butylphenoxy)-N-(4,6-dimethoxy-5-pyrimidinyl)-2,2-dimethylpentanamide, mp 94-95°C, were prepared from 3-amino-2,4-dimethoxypyrimidine and 5-(butylphenoxy)-2,2-dimethylpentanoyl chloride.
Analyzed for $C_{23}H_{33}N_3O_4$ :
Calculated : C, 66.48%; H, 8.00%; N, 10.11%;
Found : C, 66.76%; H, 7.75%; N, 10.16%

Example 8

Preparation of 5-[4-(1,1-dimethylethyl)phenoxy]-N-(2,4-dimethylphenyl)-2,2-dimethylpentanamide

Step A - Preparation of 4-(1,1-dimethylethyl)phenoxy-2,2-dimethylpentanoic acid, methyl ester.

4-(1,1-Dimethylethyl)phenol (30.0 g, 0.2 mol), 44.6 g (0.2 mol) of 5-bromo-2,2-dimethylpentanoic acid methyl ester, and 27.64 g (0.2 mol) of potassium carbonate were mixed in 300 ml of acetonitrile and the resulting mixture was stirred and heated under reflux overnight. The mixture was cooled, filtered to remove solids, and the filtrate was concentrated under vacuum.

The filtrate was washed twice with a 1 molar aquous solution of sodium hydroxide, once with brine solution, and then dried and concentrated under vacuum to yield 56.8 g of 5-[4-(1,1-dimethylethyl)phenoxy]-2,2-dimethylpentanoic acid methyl ester which was employed without further purification.

Step B - Preparation of 5-[4-(1,1-dimethylethyl)-phenoxy]-2,2-dimethylpentanoic acid.

The ester (56.8 g, 0.195 mol), prepared as described in Step A, was mixed with 290 ml of aqueous 1 molar sodium hydroxide solution and 300 ml of methanol. This mixture was heated under reflux overnight with stirring. The mixture was then cooled, concentrated under vacuum, and made acidic with concentrated hydrochloric acid. The precipitated product was collected by filtration, washed with water, and dried to yield 50.4 g of 5-[4-(1,1-dimethylethyl)phenoxy]-2,2-dimethylpentanoic acid, mp 102-103°C.

Step C - Preparation of 5-[4-(1,1-dimethylethyl)phenoxy]-2,2-dimethylpentanoyl chloride.

5-[4-(1,1-Dimethylethyl)phenoxy]-2,2-dimethylpentanoic acid (50.4 g 0.173 mol) was dissolved in 400 ml of toluene. To this mixture was added, in a thin stream with stirring, 43.9 g (0.346 mol) of oxalyl chloride dissolved in 200 ml of toluene. The mixture was stirred at room temperture overnight, and then concentrated under vacuum. Toluene was added to the residue, and the solution was again concentrated under vacuum. The residue was distilled under reduced pressure to yield 51.4 g of 5-[4-(1,1-dimethyl-ethyl)-phenoxy]-2,2-dimethylpentanoyl chloride, bp 151-155°C at 0.1 Torr.

Step D - Preparation of 5-[4-(1,1-dimethylethyl)phenoxy]-N-(2,4-dimethylphenyl)-2,2-dimethylpentanamide.

2,4-Dimethylbenzeneamine (3.64 g (0.03 mol) and 3.03 g (0.03 mol) of triethylamine were dissolved in 100 ml of tetrahydrofuran. 5-[4-(1,1-Dimethylethyl)phenoxy]-2,2-dimethylpentanoyl chloride (8.89 g (0.03 mol) from Step C, dissolved in 100 ml of tetrahydrofuran, was added to this mixture in a thin stream with stirring. The resulting mixture was stirred at room temperature overnight and then filtered. The filtrate was concentrated under vacuum to yield and oil which solidified upon scratching. Recrystallization of this solid from n-pentane yielded 9.3 g of 5-[4-(1,1-dimethylethyl)phenoxy]-N-(2,4-dimethylphenyl)-2,2-dimethylpentanamide, mp 75-76°C.
Analyzed for $C_{25}H_{35}NO_2$ :
Calculated : C, 78.69%; H, 9.24%; N, 3.67%;
Found : C, 78.81; H, 9.31%; N, 3.81%.

Example 9

Preparation of N-(2,4-difluorophenyl)-5-[4-(1,1-dimethylethyl)phenoxy]-2,2-dimethylpentanamide

Employing the methods of Example 1, 8.8 g of N-(2,4-difluorophenyl)-5-[4-(1,1-dimethylethyl)phenoxy]-2,2-dimethylpentanamide, mp 67-68°C, were prepared from 2,4-difluorobenzeneamine and 5-[4-(1,1-dimethylethyl)-phenyl]-2,2-dimethylpentanoyl chloride.
Analyzed for $C_{23}H_{29}F_2NO_2$ :
Calculated : C, 70.93%; H, 7.50%; N, 3.60%;
Found : C, 71.34%; H, 7.58%; N, 3.58%.

Example 10

Preparation of 5-[4-(1,1-dimethylethyl)phenoxy]-2,2-dimethyl-N-(2,4,6-trimethoxyphenyl)pentanamide

Employing the methods of Example 1, 9.3 g of 5-[4-(1,1-dimethylethyl)phenoxy]-2,2-dimethyl-N-(2,4,6-trimethoxyphenyl)pentanamide, mp 97-98°C, were prepared from 2,4,6-trimethoxyaniline and 5-[4-(1,1-dimethylethyl)phenyl]-2,2-dimethylpentanoyl chloride.
Analyzed for $C_{26}H_{37}NO_5$ :
Calculated : C, 70.40%, H, 8.41%; N, 3.16%;
Found : C, 70.61%; H, 8.28%; N, 3.11%.

Example 11

Preparation of N-(2,4-dimethoxyphenyl)-5-[4-(1,1-dimethylethyl)phenoxy]-2,2-dimethylpentanamide

Employing the methods of Example 1, 10.3 g of N-(2,4-dimethoxyphenyl)-5-[4-(1,1-dimethylethyl)-phenoxy]-2,2-dimethylpentanamide, mp 52-54 °C were prepared from 2,4-dimethoxybenzeneamine and 5-[4-(1,1-dimethylethyl)-phenyl]-2,2-dimethylpentanoyl chloride.
Analyzed for $C_{25}H_{35}NO_4$ :
Calculated : C, 72.61%; H, 8.53%; N, 3.39%;
Found : C, 72.61%; H, 8.38%; N, 3.33%.

Example 12

Preparation of 5-[4-(1,1-dimethylethyl)phenoxy]-2,2-dimethyl-N-[1-(2-pyridinyl)ethyl]pentanamide

Employing the methods of Example 1, 10.3 g of N-(2,4-dimethoxyphenyl)-5-[4-(1,1-dimethylethyl)-phenoxy]-2,2-dimethylpentanamide, bp 185-190°C at 0.05 Torr, were prepared from 1-(2-pyridinyl)-ethaneamine and 5-[4-(1,1-dimethylethyl)-phenyl]-2,2-dimethylpentanoyl chloride. Analyzed for $C_{24}H_{34}N_2O_2$ :
Calculated : C, 75.35%; H, 8.96%; N, 7.32%;
Found : C, 75.57%; H, 9.06%; N, 7.16%.

Example 13

Preparation of 5-[4-(1,1-dimethylpropyl)phenoxy]-2,2-dimethyl-N-[1-(2-pyridinyl)ethyl]pentanamide

Step A - Preparation of 5-[4-(1,1-dimethylpropyl)phenoxy]-2,2-dimethylpentanoic acid methyl ester.

Employing the general method of Example 1, Step A, 32.85 g (0.2 mol) of 4-(1,1-dimethylpropyl)phenol and 44.6 g (0.2 mol) of 5-bromo-2,2-dimethylpentanoic acid methyl ester were converted to 62.1 g of 5-[4-(1,1-dimethylpropyl)-phenoxy]-2,2-dimethylpentanoic acid methyl ester which was employed in the next step without further purification.

Step B - Preparation of 5-[4-(1,1-dimethylpropyl)phenoxy]-2,2-dimethylpentanoic acid

Employing the general method of Example 1, Step B, the ester was saponified to produce 52.3 g of 5-[4-(1,1-dimethylpropyl)phenoxy]-2,2-dimethylpentanoic acid, mp 83-85°C.

Step C - Preparation of 5-[4-(1,1-dimethylpropyl)phenoxy]-2,2-dimethylpentanoyl chloride

Employing the general method of Example 1, Step C above, the acid was converted to 46.0 g of 5-[4-(1,1-dimethylpropyl)-phenoxy]-2,2-dimethylpentanoyl chloride, bp 165-168°C at 0.45 torr.

Step D - Preparation of 5-[4-(1,1-dimethylpropyl)-phenoxy]-2,2-dimethyl-N-[1-(2-pyridinyl)ethyl]pentanamide

Employing the general method of Example 1, Step D above, the acid chloride was converted, by reaction with 2-(2-pyridinyl)ethaneamine to 10.4 g of 5-[4-(1,1-dimethyl-propyl)phenoxy]-2,2-dimethyl-N-[1-(2-pyridinyl)ethyl]pentan-amide, bp 186-190°C at 0.1 torr.
Analyzed for $C_{25}H_{30}N_2O_2$ :
Calculated : C, 75.72%; H, 9.13%; N, 7.06%;
Found : C, 75.75%; H, 8.83%; N, 6.91%.

Example 14

Preparation of 5-[4-(1,1-dimethylpropyl)phenoxy]-2,2-dimethyl-N-(2,4,6-trimethoxyphenyl)pentanamide

Employing the methods of Example 13, 7.6 g of 5-[4-(1,1-dimethylpropyl)phenoxy]-2,2-dimethyl-N--(2,4,6-trimethoxyphenyl)pentanamide, mp. 85-87°C, were prepared from 2,4,6-trimethoxybenzeneamine and 5-(1,1-dimethylpropyl-phenoxy)-2,2-dimethylpentanoyl chloride.
Analyzed for $C_{27}H_{39}NO_5$ :
Calculated : C, 70,87%; H, 8.59%; N, 3.06%;
Found : C, 70.98%; H, 8.39%; N, 3.00%.

Example 15

Preparation of 5-(4-hexylphenoxy)-2,2-dimethyl-N-[1-(2-pyridinyl)ethyl]pentanamide

Step A - Preparation of 5-(4-hexylphenoxy)-2,2-dimethylpentanoic acid methyl ester

Employing the general method of Example 1, Step A, 42.6g of 5-(4-hexylphenoxy)-2,2-dimethylpentanoic acid methyl ester were prepared from 4-hexylphenol and 5-bromo-2,2-dimethylpentanoic acid. The material was used in the succeeding step without further purification.

Step B - Preparation of 5-(4-hexylphenoxy)-2,2-dimethylpentanoic acid

Employing the method of Example 1, Step B, the ester was sponified to produce 36.9 g of 5-(4-hexylphenoxy)-2,2-dimethylpentanoic acid, mp 38-40°C by the action of aqueous sodium hydroxide.

Step C - Preparation of 5-(4-hexylphenoxy)-2,2-dimethylpentanoyl chloride

Employing the method of Example 1, Step C above, the acid was converted by the action of oxalyl chloride to 33.2 g of 5-(4-hexylphenoxy)-2,2-dimethylpentanoyl chloride, bp 150-160°C at 0.1-0.2 torr.

Step D - Preparation of 5-(4-hexylphenoxy)-2,2-dimethyl-N-[1-(2-pyridinyl)ethyl]pentanamide

Employing the method of Example 1, Step D, the acid chloride was reacted with 1-(2-pyridinyl)-ethaneamine to produce 9.7 g of 5-(4-hexylphenoxy)-2,2-dimethyl-N-[1-(2-pyridinyl)ethyl]pentanamide.
Analyzed for $C_{26}H_{38}N_2O_2$ :
Calculated : C, 76.06%; H, 9.33%; N, 6.82%;
Found : C, 76.15%, H, 9.08%; N, 6.82%

0.92 (m, 3H), 1.15 (bs, 14H), 1.28 (d, 3H, J = 7Hz), 1.70 (s, 4H), 2.55 (brt, 2H, 7Hz), 3.9 (m, 2H), 5.20 (m, 1H), 6.8-7.3 (m, 7H), 7.6 (m, 1H), 8.5 (m, 1H)

Example 16

Preparation of 2,2-dimethyl-5-(4-octylphenoxy)-N-(2,4,6-trimethoxyphenyl)pentanamide

Step A - Preparation of 5-(4-octylphenoxy)-2,2-dimethylpentanoic acid methyl ester

Employing the method of Example 1, Step A, 49.7 g of 5-(4-octylphenoxy)-2,2-dimethylpentanoic acid methyl ester were prepared and employed in the next step without futher purification.

Step B - Preparation of 5-(4-octylphenoxy)-2,2-dimethylpentanoic acid

The ester was saponified by the action of aqueous sodium hydroxide employing the method of Example 1, Step B to produce 42.7 g of 5-(4-octylphenoxy)-2,2-dimethylpentanoic acid as a white wax. This material was employed in the next step without further purification.

Step C - Preparation of 5-(4-octylphenoxy)-2,2-dimethyl-pentanoyl chloride

Employing the method of Example 1, Step C, 45.5 g of 5-(4-octylphenoxy)-2,2-dimethylpentanoyl chloride.

Step D - Preparation of 2,2-dimethyl-5-(4-octylphenoxy)-N-(2,4,6-trimethoxyphenyl)pentanamide

Employing the method of Example 1, Step D, 2,4,6-trimethoxybenzeneamine was reacted with 5-(4-octylphenoxy)-2,2-dimethylpentanoyl chloride to produce 4.1 g of 2,2-dimethyl-5-(4-octylphenoxy)-N-(2,4,6-trimethoxyphenyl)-pentanamide, mp 67-68°C.
Analyzed for $C_{30}H_{45}NO_5$ :
Calculated : C, 72.11%; H, 9.08%; N, 2.80%;
Found : C, 71.80%; H, 8.96%; N, 2.73%.

Example 17

Preparation of 2,2-dimethyl-5-(4-octylphenoxy)-N-[1-(2-pyridinyl)ethyl]pentanamide

Employing the methods of Example 16, 5.0 g of 2,2-dimethyl-5-(4-octylphenoxy)-N-[1-(2-pyridinyl)-ethyl]-pentanamide were prepared by reacting 1-(2-pyrindyl)-ethaneamine with 5-(4-octylphenoxy)-2,2-dimethylpentanoyl chloride.
Analyzed for $C_{28}H_{42}N_2O_2$ :
Calculated: C, 76.67%; H, 9.65%; N, 6.39%;
Found : C, 76.87%; H, 9.65%; N, 6.42%.

100 mHz NMR ($CDCl_3$), 0.95 (m, 3H), 1.3 (m, 18H), 1.45 (d, 3H, J = 7Hz), 1.70 (m, 4H), 2.55 (m, 2H), 3.9 (m, 2H), 5.1 (septet, 1H, J = 7Hz), 6.7-7.3 (m, 7H), 7.7 (m, 1H), 8.6 (m, 1H)

## Claims

1. A compound having the formula I

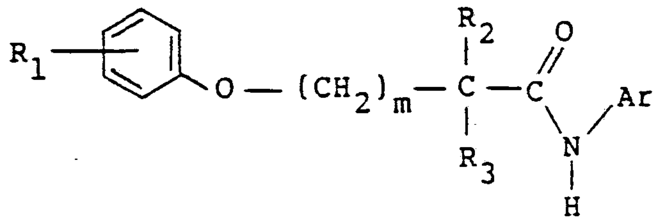

(I),

where $R_1$ is straight or branched alkyl of from one to nine carbon atoms, cycloalkyl of from three to seven carbon atoms, or alkyloxy of from one to six carbon atoms; and m is an integer of from one to six; $R_2$ and $R_3$ are independently selected from hydrogen, straight or branched alkyl of from one to six carbon atoms, or, when taken together with the carbon atom to which they are attached, form a cyclopropyl ring; Ar is

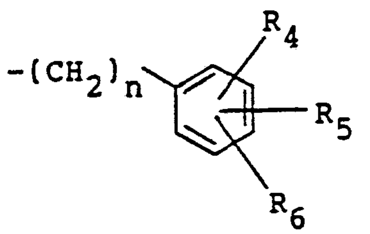

,

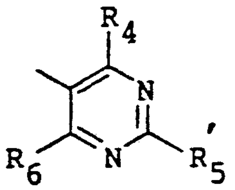

,

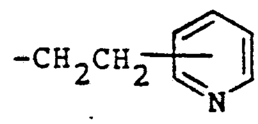

,

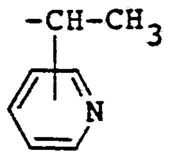

, or

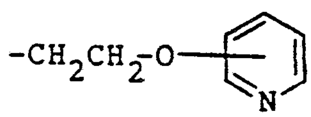

where n is zero or one, $R_4$, $R_5$ and $R_6$ are independently straight or branched alkyl of from one to three carbon atoms, straight or branched alkyloxy of from one to three carbon atoms, F, Cl, Br, or carboalkoxy of from two to six carbon atoms with the proviso that when Ar is

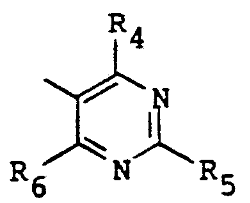

,

$R_4$, $R_5$, and $R_6$ may not be F, Cl, or Br.

2. A compound as defined in Claim 1 wherein $R_1$ is alkyl.

3. A compound as defined in Claim 1 or 2 selected from the group consisting of 5-[4-(1,1-dimethylethyl)phenoxy]-N-(2,4-dimethylphenyl)-2,2-dimethylpentanamide,
N-(2,4-difluorophenyl)-5-[4-(1,1-dimethylethyl)phenoxy]-2,2-dimethylpentanamide,
5-(4-butylphenoxy)-N-(2-methoxy-4-methylphenyl)-2,2-dimethylpentanamide,
5-(4-butylphenoxy)-N-(2-methoxyphenyl)-2,2-dimethylpentanamide,
N -(2,4-dimethoxyphenyl)-5-[4-(1,1-dimethylethyl)phenoxy]-2,2-dimethylpentanamide,
5-(4-butylphenoxy)-2,2-dimethyl-N-(2,4,6-trimethoxyphenyl)pentanamide,
5-[4-(1,1-dimethylethyl)phenoxy]-2,2-dimethyl-N-(2,4,6-trimethoxyphenyl)pentanamide,
2,2-dimethyl-5-(4-octylphenoxy)-N-(2,4,6-trimethoxyphenyl)pentanamide,
2,2-dimethyl-5-(4-methylphenoxy)-N-[1-(2-pyridinyl)ethyl]pentanamide,

5-(4-butylphenoxy)-2,2-dimethyl-N-[1-(2-pyridinyl)ethyl]pentanamide,
5-[4-(1,1-dimethylethyl)phenoxy]-2,2-dimethyl-N-[1-(2-pyridinyl)ethyl]pentanamide,
5-[4-(1,1-dimethylpropyl)phenoxy]-2,2-dimethyl-N-[1-(2-pyridinyl)ethyl]pentanamide,
5-(4-hexylphenoxy)-2,2-dimethyl-N-[1,(2-pyridinyl)ethyl]pentanamide,
2,2-dimethyl-5-(4-octylphenoxy)-N-[1-(2-pyridinyl)ethyl]pentanamide,
5-(4-butylphenoxy-2,2-dimethyl-N-[2-(2-pyridinyloxy)ethyl]pentanamide and
5-(4-butylphenoxy)-N-(4,6-dimethoxy-5-pyrimidinyl)-2,2-dimethylpentanamide.

4. A pharmaceutical composition comprising a compound as defined by Claims 1 to 3 in combination with a pharmaceutically acceptable carrier.

5. Use of a compound as defined in claims 1 to 3 for the manufacture of a pharmaceutical for inhibiting intestinal absorption of cholesterol.

6. A process of preparing a compound having the structural formula

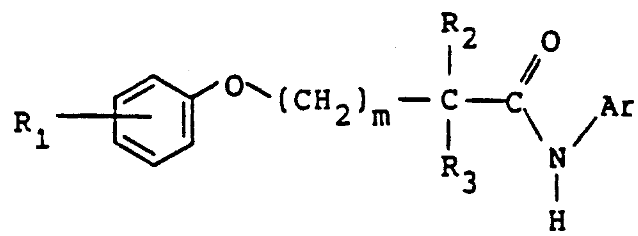

where $R_1$ is straight or branched alkyl of from one to nine carbon atoms, cycloalkyl of from three to seven carbon atoms, or alkyloxy of from one to six carbon atoms; m is an integer of from one to six; $R_2$ and $R_3$ are independently selected from hydrogen, straight or branched alkyl of from one to six carbon atoms, or, when taken together with the carbon atom to which they are attached, form a cyclopropyl ring;
Ar is

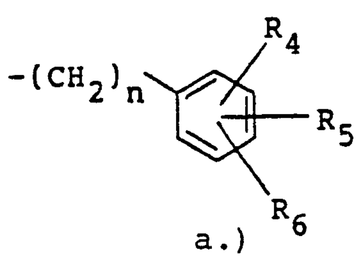 , 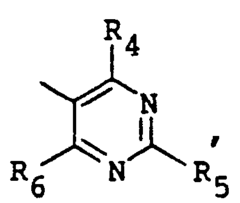 , 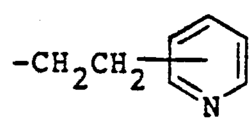 ,

a.) b.) c.)

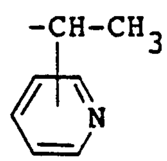 , or 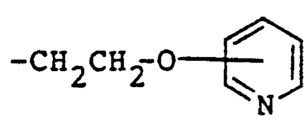

d.) e.)

where n is zero or one, $R_4$, $R_5$, and $R_6$ are independently straight or branched alkyl of from one to three carbon atoms, straight or branched alkyloxy of from one to three carbon atoms, F, Cl, Br, or carboalkoxy of from two to six carbon atoms; with the proviso that when Ar is

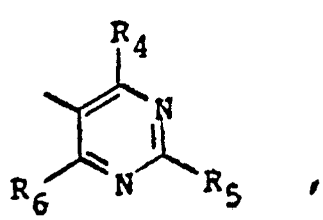 ,

$R_4$, $R_5$, and $R_6$ may not be F, Cl, or Br; comprising reacting an acid chloride of the formula II

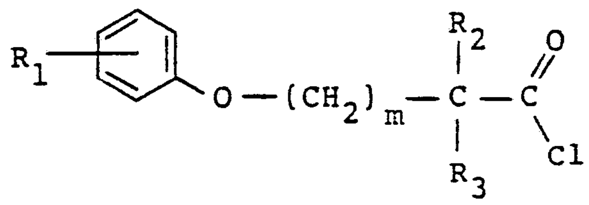 (II),

where m, X, and $R_1$ are as defined above, with an amine selected from the group consisting of

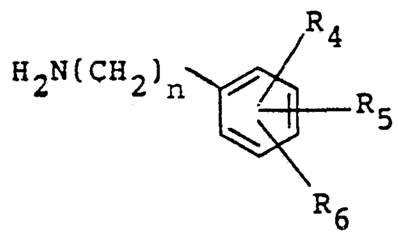 , 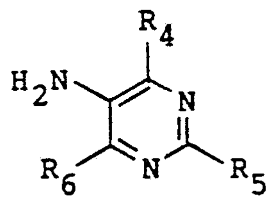 , 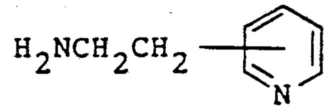 ,

a.) b.) c.)

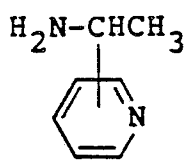 , or 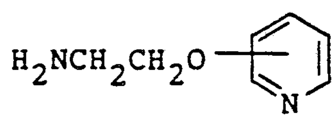 .

d.) e.)

where n $R_4$, $R_5$, and $R_6$ are as defined above, and further converting the product of the reaction.

Claims for the following contracting states: AT, ES, GR.

1. A process of preparing a compound having the structural formula I

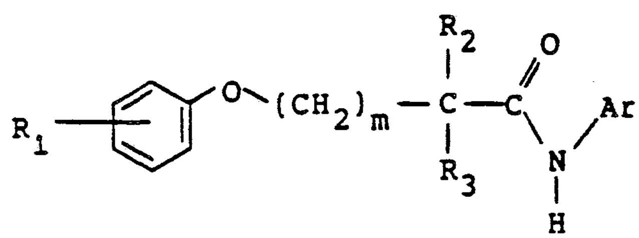 (I),

where $R_1$ is straight or branched alkyl of from one to nine carbon atoms, cycloalkyl of from three to seven carbon atoms, or alkyloxy of from one to six carbon atoms; and m is an integer of from one to six; $R_2$ and $R_3$ are independently selected from hydrogen, straight or branched alkyl of from one to six carbon atoms, or, when taken together with the carbon atom to which they are attached, form a cyclopropyl ring;
Ar is

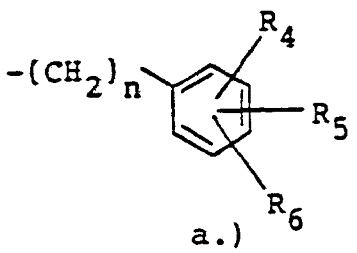 , 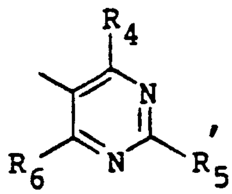 , 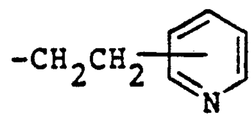 ,

a.) b.) c.)

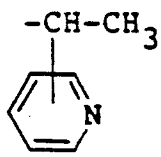 , or 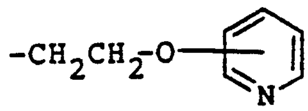

d.) e.)

where n is zero or one, $R_4$, $R_5$ and $R_6$ are independently straight or branched alkyl of from one to three carbon atoms, straight or branched alkyloxy of from one to three carbon atoms, F, Cl, Br, or carboalkoxy of from two to six carbon atoms; with the proviso that when Ar is

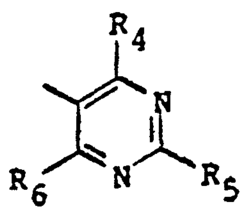 ,

$R_4$, $R_5$, and $R_6$ may not be F, Cl, or Br; comprising reacting an acid chloride of the formula II

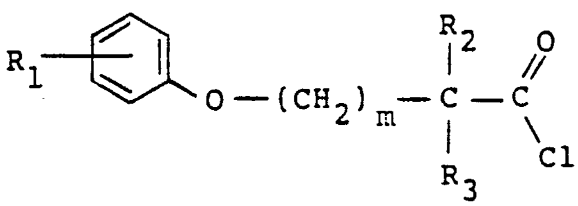 (II),

where m, X, and $R_1$ are as defined above, with an amine selected from the group consisting of

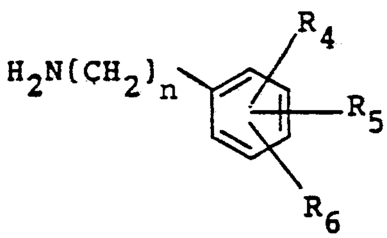 , 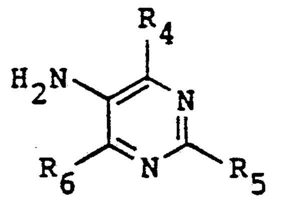 , 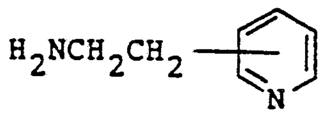

a.) b.) c.)

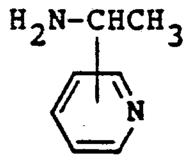 , or 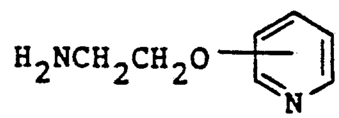 .

d.) e.)

where n $R_4$, $R_5$, and $R_6$ are as defined above.

2. A process as defined in Claim 1 wherein $R_1$ is alkyl.

3. A process as defined in Claim 1 or 2 for the manufacture of a compound selected from the group consisting of 5-[4-(1,1-dimethylethyl)phenoxy]-N-(2,4-dimethylphenyl)-2,2-dimethylpentanamide,
N-(2,4-difluorophenyl)-5-[4-(1,1-dimethylethyl)phenoxy]-2,2-dimethylpentanamide,
5-(4-butylphenoxy)-N-(2-methoxy-4-methylphenyl)-2,2-dimethylpentanamide,
5-(4-butylphenoxy)-N-(2-methoxyphenyl)-2,2-dimethylpentanamide,
N -(2,4-dimethoxyphenyl)-5-[4-(1,1-dimethylethyl)-phenoxy]-2,2-dimethylpentanamide,
5-(4-butylphenoxy)-2,2-dimethyl-N-(2,4,6-trimethoxyphenyl)pentanamide,
5-[4-(1,1-dimethylethyl)phenoxy]-2,2-dimethyl-N-(2,4,6-trimethoxyphenyl)pentanamide,
2,2-dimethyl-5-(4-octylphenoxy)-N-(2,4,6-trimethoxyphenyl)pentanamide,
2,2-dimethyl-5-(4-methylphenoxy)-N-[1-(2-pyridinyl)ethyl]pentanamide,
5-(4-butylphenoxy)-2,2-dimethyl-N-[1-(2-pyridinyl)ethyl]pentanamide,
5-[4-(1,1-dimethylethyl)phenoxy]-2,2-dimethyl-N-[1-(2-pyridinyl)ethyl]pentanamide,
5-[4-(1,1-dimethylpropyl)phenoxy]-2,2-dimethyl-N-[1-(2-pyridinyl)ethyl]pentanamide,
5-(4-hexylphenoxy)-2,2-dimethyl-N-[1,(2-pyridinyl)ethyl]pentanamide,
2,2-dimethyl-5-(4-octylphenoxy)-N-[1-(2-pyridinyl)ethyl]pentanamide,
5-(4-butylphenoxy-2,2-dimethyl-N-[2-(2-pyridinyloxy)ethyl]pentanamide and
5-(4-butylphenoxy)-N-(4,6-dimethoxy-5-pyrimidinyl)-2,2-dimethylpentanamide.

4. Use of a compound manufactured according to claims 1 to 3 for the manufacture of a pharmaceutical for inhibiting intestinal absorption of cholesterol.